# EUROPEAN PATENT SPECIFICATION

(11) **EP 3 080 606 B1**
(45) Date of publication and mention of the grant of the patent: **28.08.2019**
(21) Application number: 14812183.3
(22) Date of filing: 09.12.2014
(51) Int. Cl.: G01N 33/543, G01N 33/68

(54) **SPR-BASED BRIDGING ASSAY FOR DETERMINING THE BIOLOGICAL ACTIVITY OF MULTIVALENT, MULTISPECIFIC MOLECULES**
OBERFLÄCHEN-PLASMONRESONANZ BASIERTER ÜBERBRÜCKUNGSTEST ZUR BESTIMMUNG DER BIOLOGISCHEN AKTIVITÄT VON MULTIVALENTEN, MULTISPEZIFISCHEN MOLEKÜLEN
DOSAGE PONTÉ BASÉ SUR LA RÉSONANCE PLASMONIQUE DE SURFACE POUR DÉTERMINER L'ACTIVITÉ BIOLOGIQUE DE MOLÉCULES POLYSPÉCIFIQUES ET POLYVALENTES

(30) Priority: 13.12.2013 EP 13197264
(43) Date of publication of application: 19.10.2016
(73) Proprietor: F. Hoffmann-La Roche AG, 4070 Basel (CH)
(72) Inventor: PAPADIMITRIOU, Apollon, 83673 Bichl (DE); REGULA, Joerg Thomas, 81377 Muenchen (DE); KETTENBERGER, Hubert, 81369 Muenchen (DE); MOELLEKEN, Joerg, 80686 Muenchen (DE); GASSNER, Christian, 82377 Penzberg (DE)
(74) Representative: Burger, Alexander
(86) International application number: PCT/EP2014/076953
(87) International publication number: WO 2015/086549

(56) References cited:
- WO-A1-2010/040508
- WO-A1-2011/117329
- SCHAEFER W. ET AL.: "Immunoglobulin domain crossover as a generic approach for the production of bispecific IgG antibodies", PROC. NATL. ACAD. SCI. USA, vol. 108, no. 27, 5 July 2011 (2011-07-05) , pages 11187-11192, XP055003817,
- GASSNER C. ET AL.: "Development and validation of a novel SPR-based assay principle for bispecific molecules", J. PHARM. BIOMED. ANAL., vol. 102, 17 September 2014 (2014-09-17), pages 144-149, XP55168364,

## Description

Herein is reported an improved setup of a bridging assay for the determination of the biological activity of multispecific, multivalent, e.g. bispecific, bivalent, molecules in which the specificity and robustness of the assay in the presence of oligomeric side-products is increased.

### Background of the Invention

Assays for determining the biological activity of monospecific antibodies are well known. Different assay setups are known, such as e.g.
capture of the antibody via immobilized antigen (directly or indirectly immobilized to a solid phase) and detection of the antigen-antibody complex by a labeled anti-Fc-antibody, or
capture of the antigen via immobilized antibody (directly or indirectly immobilized to a solid phase) and detection of the antibody-antigen complex by a labeled antibody that binds to a different non-overlapping epitope on the antigen.

A biological activity assay is an analytical procedure for measuring the biological activity of a test substance based on a specific, functional biological response of a test system. Biological activity may be assessed using animal based in-vivo assays, cell based in-vitro assays or (receptor) binding assays.

Surface plasmon resonance (SPR) is a well know technology for the determination of antibody-antigen interactions of monospecific antibodies. The technology can be used for the determination of binding affinities of antibodies to their respective antigens.

In WO 2011/117329 bispecific, bivalent anti-VEGF/anti-ANG2 antibodies are reported.

In WO 2009/080251 bivalent, bispecific antibodies are reported. Bispecific antibodies are reported in US 2011/0293613. In WO 2010/040508 bispecific anti-VEGF/anti-ANG2 antibodies are reported. Immunoglobulin domain crossover as a generic approach for the production of bispecific IgG antibodies is reported by Schaefer, W., et al. (Proc. Natl. Acad. Sci. USA 108 (2011) 11187-11192).

### Summary of the Invention

It has been found that assays for determining the biological activity using a general principle as outlined above and not taking into account the findings as reported herein are sensitive to "functional" multimers, i.e. dimers, trimers, tetramers and higher order aggregates, of multivalent, multispecific antibodies, such as bivalent, bispecific antibodies, due to avidity based binding of the multimers.

One aspect as reported herein is the (in vitro) use of the binding site of a multivalent, multispecific antibody that has the smaller (lower) k_{D} value (dissociation constant) for the interaction with its antigen for the immobilization of the multispecific, multivalent antibody to a solid surface for the determination of the biological activity, i.e. functional binding to an antigen, of the multivalent, multispecific antibody.

In one embodiment the determination of the biological activity is by determining the (bridging) binding signal with the antigen for which the multivalent, multispecific antibody has the bigger (higher) k_{D} value (dissociation constant).

In one embodiment the biological activity is the binding affinity.

In one embodiment the determination of the (bridging) binding signal is by surface plasmon resonance or ELISA.

In one embodiment the multivalent, multispecific antibody is selected from a bivalent, bispecific antibody, a trivalent, trispecific antibody, a tetravalent, tetraspecific antibody, a trivalent, bispecific antibody, and a tetravalent, trispecific antibody. In one embodiment the multivalent, multispecific antibody is a bivalent, bispecific antibody.

One aspect as reported herein is the use of the simultaneous binding of a bivalent, bispecific antibody to its first and second antigen in a surface plasmon resonance or ELISA method, whereby the first antigen is immobilized as capture reagent on a solid surface, and whereby the bivalent, bispecific antibody has a kD value for the interaction with the first antigen that is smaller than the kD value for the interaction with the second antigen, for reducing the interference of functional multimeric forms of the bivalent, bispecific antibody in the surface plasmon resonance or ELISA method.

In one embodiment the solid surface is a surface plasmon resonance chip.

In one embodiment the first antigen is a dimer or trimer or tetramer.

In one embodiment functional multimer is a functional dimer.

In one preferred embodiment the first antigen is a dimer or trimer or tetramer and the functional multimer is a functional dimer.

In one embodiment the k_{D} value of the binding site that has the higher k_{D} value for the interaction with its antigen is at least 1.1 times higher than the k_{D} value of the binding site of the bivalent, bispecific antibody that has the smaller k_{D} value.

In one embodiment the k_{D} value is at least 10 times higher.

In one embodiment the k_{D} value is at least 100 times higher.

One aspect as reported herein is the (in vitro) use of the (bridging signal of the) simultaneous binding of a multivalent, multispecific antibody to its first and second antigen for the determination of the biological activity, i.e. functional binding to an antigen, of the multivalent, multispecific antibody,
whereby the biological activity of the multivalent, multispecific antibody is derived from the (bridging) binding signal obtained with its second antigen,
whereby the (bridging) binding signal with the second antigen is determined by surface plasmon resonance or ELISA with the first immobilized antigen as capture reagent on a solid surface, and
whereby the multivalent, multispecific antibody has a k_{D} value (dissociation constant) for the interaction with the first antigen that is smaller than the k_{D} value (dissociation constant) for the interaction with the second antigen.

In one embodiment the multivalent, multispecific antibody is selected from a bivalent, bispecific antibody, a trivalent, trispecific antibody, a tetravalent, tetraspecific antibody, a trivalent, bispecific antibody, and a tetravalent, trispecific antibody. In one embodiment the multivalent, multispecific antibody is a bivalent, bispecific antibody.

In one embodiment the determination of the (bridging) binding signal is by surface plasmon resonance.

One aspect as reported herein is the (in vitro) use of the simultaneous binding of a multivalent, multispecific antibody to its first and second antigen for the determination of the biological activity, i.e. simultaneous functional binding to the first and second antigen, of the multivalent, multispecific antibody to reduce interference from multimeric forms of the multivalent, multispecific antibody,
whereby the biological activity of the multivalent, multispecific antibody is derived from the (bridging) binding signal obtained with its second antigen,
whereby the (bridging) binding signal with the second antigen is determined by surface plasmon resonance or ELISA with the first immobilized antigen as capture reagent on a solid surface, and
whereby the multivalent, multispecific antibody has a k_{D} value (dissociation constant) for the interaction with the first antigen that is smaller than the k_{D} value (dissociation constant) for the interaction with the second antigen.

In one embodiment the multivalent, multispecific antibody is selected from a bivalent, bispecific antibody, a trivalent, trispecific antibody, a tetravalent, tetraspecific antibody, a trivalent, bispecific antibody, and a tetravalent, trispecific antibody. In one embodiment the multivalent, multispecific antibody is a bivalent, bispecific antibody.

In one embodiment the determination of the (bridging) binding signal is by surface plasmon resonance.

In one embodiment of all previous aspects and embodiment the determining of the binding signal for the simultaneous binding of the multivalent, multispecific antibody to its first and second antigen is based on the binding of the bivalent, bispecific antibody to its second antigen.

In one embodiment of all previous aspects and embodiments the solid surface is a surface plasmon resonance chip or the wall of a well of a multi-well plate.

In one embodiment of all previous aspects and embodiments the k_{D} value (dissociation constant) of the binding site that has the bigger (higher) k_{D} value (dissociation constant) for the interaction with its antigen is at least 1.1 times bigger (higher) than the k_{D} value (dissociation constant) of the binding site of the multivalent, multispecific antibody that has the smaller (lower) k_{D} value (dissociation constant). In one embodiment the k_{D} value (dissociation constant) is at least 1.5 times bigger (higher). In one embodiment the k_{D} value (dissociation constant) is at least 2 times bigger (higher). In one embodiment the k_{D} value (dissociation constant) is at least 10 times bigger (higher). In one embodiment the k_{D} value (dissociation constant) is at least 100 times bigger (higher).

In one embodiment of all previous aspects and embodiments the first antigen is a dimer or trimer or tetramer or prone to aggregation.

In one embodiment of all previous aspects and embodiments the functional multimer is a functional dimer.

One aspect as reported herein is an (in vitro) method for determining the biological activity, i.e. functional binding to an antigen, of a multivalent, multispecific antibody comprising the step of:
- determining the (bridging) binding signal for the simultaneous binding of the multivalent, multispecific antibody to its first and second antigen,
whereby the biological activity of the multivalent, multispecific antibody is derived from the (bridging) binding signal obtained with its second antigen,
whereby the (bridging) binding signal with the second antigen is determined by surface plasmon resonance or ELISA with the first immobilized antigen as capture reagent on a solid surface, and
whereby the multivalent, multispecific antibody has a k_{D} value (dissociation constant) for the interaction with the first antigen that is smaller than the k_{D} value (dissociation constant) for the interaction with the second antigen.

In one embodiment the determination of the (bridging) binding signal is by surface plasmon resonance.

In one embodiment the multivalent, multispecific antibody is selected from a bivalent, bispecific antibody, a trivalent, trispecific antibody, a tetravalent, tetraspecific antibody, a trivalent, bispecific antibody, and a tetravalent, trispecific antibody. In one embodiment the multivalent, multispecific antibody is a bivalent, bispecific antibody.

One aspect as reported herein is an (in vitro) method for reducing the interference from functional multimeric forms of a multivalent, multispecific antibody in the determination of the biological activity, i.e. functional binding, of the multivalent, multispecific antibody comprising the step of:
- determining the (bridging) binding signal for the simultaneous binding of the multivalent, multispecific antibody to its first and second antigen,
whereby the biological activity of the multivalent, multispecific antibody is derived from the (bridging) binding signal obtained with its second antigen,
whereby the (bridging) binding signal with the second antigen is determined by surface plasmon resonance or ELISA with the first immobilized antigen as capture reagent on a solid surface, and
whereby the multivalent, multispecific antibody has a k_{D} value (dissociation constant) for the interaction with the first antigen that is smaller than the k_{D} value (dissociation constant) for the interaction with the second antigen.

In one embodiment the determination of the (bridging) binding signal is by surface plasmon resonance.

In one embodiment the multivalent, multispecific antibody is selected from a bivalent, bispecific antibody, a trivalent, trispecific antibody, a tetravalent, tetraspecific antibody, a trivalent, bispecific antibody, and a tetravalent, trispecific antibody. In one embodiment the multivalent, multispecific antibody is a bivalent, bispecific antibody.

One aspect as reported herein is an (in vitro) method for determining the presence of functional multimers of a multivalent, multispecific antibody in a sample, comprising the step of
- comparing the biological activity, i.e. binding signal, determined for the multivalent, multispecific antibody using an assay wherein a first antigen is immobilized and used for the capture of the multivalent, multispecific antibody with the biological activity, i.e. binding signal, determined for the multivalent, multispecific antibody using the same assay wherein the second antigen is immobilized and used for the capture of the multivalent, multispecific antibody,
whereby the presence of functional multimers of the multivalent, multispecific antibody is determined if the determined biological activities, i.e. binding signals, differ by more than the standard deviation of the performed assay.

In one embodiment the determination of the (bridging) binding signal is by surface plasmon resonance.

In one embodiment the multivalent, multispecific antibody is selected from a bivalent, bispecific antibody, a trivalent, trispecific antibody, a tetravalent, tetraspecific antibody, a trivalent, bispecific antibody, and a tetravalent, trispecific antibody. In one embodiment the multivalent, multispecific antibody is a bivalent, bispecific antibody.

In one embodiment of all previous aspects and embodiments that the determining the binding signal for the simultaneous binding of the bivalent, bispecific antibody to its first and second antigen is based on the binding of the bivalent, bispecific antibody to its second antigen.

In one embodiment of all previous aspects and embodiments the solid surface is a surface plasmon resonance chip.

In one embodiment of all previous aspects and embodiments the first antigen is a dimer or trimer or tetramer or prone to aggregation.

In one embodiment of all previous aspects and embodiments the functional multimer is a functional dimer.

### Detailed Description of the Invention

It has been found that assays for determining the biological activity using a principle not taking into account the findings as reported herein are sensitive to "functional" multimers, i.e. functional dimers, functional trimers, functional tetramers and functional higher order aggregates, of multivalent, multispecific antibodies due to avidity based binding of the multimers.

The term "antibody" herein is used in the broadest sense and encompasses various antibody structures, including but not limited to monoclonal antibodies and multispecific antibodies (e.g., bispecific antibodies).

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

In certain embodiments, the antibody is a multispecific antibody, e.g. a bispecific antibody. Multispecific antibodies are monoclonal antibodies that have binding specificities for at least two different sites. In certain embodiments, one of the binding specificities is for a first antigen and the other is for a different second antigen. In certain embodiments, multispecific antibodies may bind to two different epitopes of the same antigen. Multispecific antibodies may also be used to localize cytotoxic agents to cells which express the antigen. Multispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A., et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (WO 2009/089004); cross-linking two or more antibodies or fragments (see, e.g., US 4,676,980, and Brennan, M., et al., Science 229 (1985) 81-83); using leucine zippers to produce bi-specific antibodies (see, e.g., Kostelny, S.A., et al., J. Immunol. 148 (1992) 1547-1553; using "diabody" technology for making bispecific antibody fragments (see, e.g., Holliger, P., et al., Proc. Natl. Acad. Sci. USA 90 (1993) 6444-6448); and using single-chain Fv (sFv) dimers (see, e.g., Gruber, M., et al., J. Immunol. 152 (1994) 5368-5374); and preparing trispecific antibodies as described, e.g., in Tutt, A., et al., J. Immunol. 147 (1991) 60-69).

The antibody or fragment can also be a multispecific antibody as described in WO 2009/080251, WO 2009/080252, WO 2009/080253, WO 2009/080254, WO 2010/112193, WO 2010/115589, WO 2010/136172, WO 2010/145792, or WO 2010/145793.

Bispecific antibodies are generally antibody molecules that specifically bind to two different, non-overlapping epitopes on the same antigen or to two epitopes on different antigens.

Different bispecific antibody formats are known.

Exemplary bispecific antibody formats for which the methods as reported herein can be used are
- the Crossmab format (= Crossmab): full length IgG antibody comprising a first binding site that specifically binds to a first epitope or antigen and a second binding site that specifically binds to a second epitope or antigen, whereby the individual chains are as follows
   - light chain 1 (variable light chain domain + light chain kappa constant domain)
   - light chain 2 (variable light chain domain + heavy chain CH1 domain)
   - heavy chain 1 (variable heavy chain domain + CH1 + Hinge + CH2 + CH3 with hole mutation)
   - heavy chain 2 (variable heavy chain domain + light chain kappa constant domain + Hinge + CH2 + CH3 with knob mutation);
- the one-armed single chain format (= one-armed single chain antibody): antibody comprising a first binding site that specifically binds to a first epitope or antigen and a second binding site that specifically binds to a second epitope or antigen, whereby the individual chains are as follows
   - light chain (variable light chain domain + light chain kappa constant domain)
   - combined light/heavy chain (variable light chain domain + light chain kappa constant domain + G₄S-Linker + variable heavy chain domain + CH1 + Hinge + CH2 + CH3 with knob mutation)
   - heavy chain (variable heavy chain domain + CH1 + Hinge + CH2 + CH3 with hole mutation);
- the two-armed single chain format (= two-armed single chain antibody): antibody comprising a first binding site that specifically binds to a first epitope or antigen and a second binding site that specifically binds to a second epitope or antigen, whereby the individual chains are as follows
   - combined light/heavy chain 1 (variable light chain domain + light chain kappa constant domain + G₄S-Linker + variable heavy chain domain + CH1 + Hinge + CH2 + CH3 with hole mutation)
   - combined light/heavy chain 2 (variable light chain domain + light chain kappa constant domain + G₄S-Linker + variable heavy chain domain + CH1 + Hinge + CH2 + CH3 with knob mutation);
- the common light chain bispecific format (= common light chain bispecific antibody): antibody comprising a first binding site that specifically binds to a first epitope or antigen and a second binding site that specifically binds to a second epitope or antigen, whereby the individual chains are as follows
   - light chain (variable light chain domain + light chain kappa constant domain)
   - heavy chain 1 (variable heavy chain domain + CH1 + Hinge + CH2 + CH3 with hole mutation)
   - heavy chain 2 (variable heavy chain domain + CH1 + Hinge + CH2 + CH3 with knob mutation).

In one embodiment the bivalent, bispecific antibody is a Crossmab.

In one embodiment the bivalent, bispecific antibody is a one-armed single chain antibody.

In one embodiment the bivalent, bispecific antibody is a two-armed single chain antibody.

In one embodiment the bivalent, bispecific antibody is a common light chain bispecific antibody.

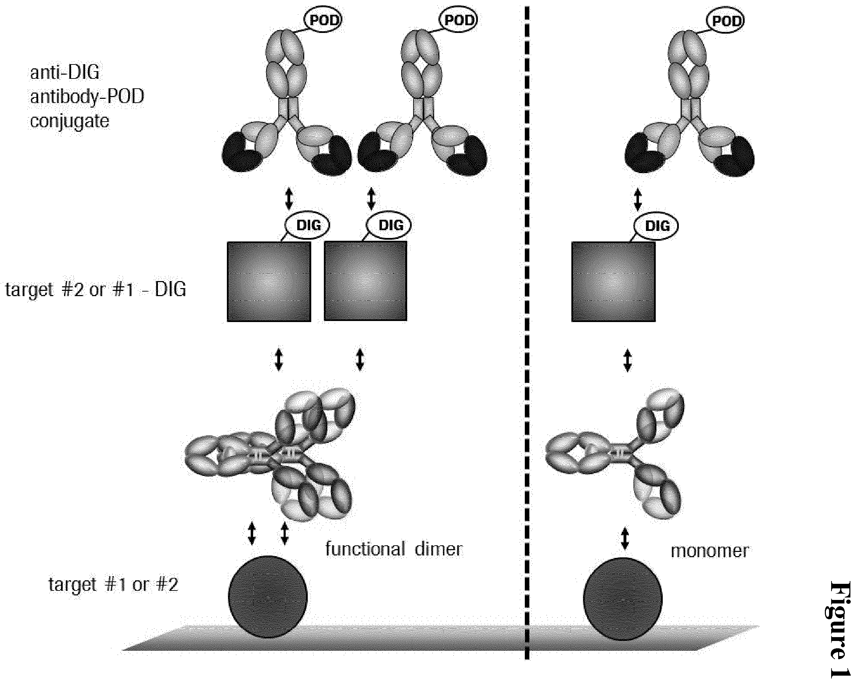

The term "functional multimer" denotes a non-covalent complex or aggregate of multivalent, multispecific antibodies in which each of the multivalent, multispecific antibody molecules maintains its binding specificities, i.e. can still bind to its antigens. For example, a "functional dimer" is a non-covalent complex consisting of two molecules of the multivalent, multispecific antibody in which each of the binding sites maintains its binding specificity and binding ability, i.e. for example in total a functional dimer of a bivalent, bispecific antibody comprises four binding sites of which each two bind to the same antigen and all four binding sites are functional. But, if an antibody is present as functional multimer all antibody molecules can bind to immobilized antigen molecules (used to capture the multivalent, multispecific antibody) at the same time. This increases the complex stability of the functional multimer as compared to the monomeric molecule, and in turn the more unstable monomeric molecules might dissociate during the assay procedure. This results in that a significantly reduced amount of the monomeric molecule is present relative to the initial condition of the sample. As the detection of the complex is based on the interaction with a second binding specificity of the multivalent, multispecific antibody that it not used for the capturing of the antibody a captured immobilized functional dimer will result in an increased assay readout (see Figure 1).

For example a change of the fraction of functional multimers (dimers) in a tested antibody preparation results in different assay readouts if the more unstable complex interaction is immobilized. Thus, assays for determining the biological activity of multivalent, multispecific antibodies are sensitive to avidity-driven apparent increase of the biological activity.

An analytical procedure for measuring the biological activity of a test substance is based on a specific, functional biological response of a test system.

The term "biological assay" denotes an analytical method for determining the biological activity of a test substance, e.g. of an antibody, based on a specific, functional biological response of a test system. Biological activity may be assessed using animal based in-vivo assays, cell based in-vitro assays, or (receptor) binding assays. In one embodiment the term "biological activity" denotes the binding to the test substance to its interaction partner. In case of a bispecific antibody the term "biological activity" denotes the binding to the antibody to its respective antigens, either determined individually, i.e. separately for each antigen, or simultaneously, i.e. for both antigens concomitantly in a bridging assay.

Specificity is the ability to assess unequivocally the analyte in the presence of interfering components which may be expected to be present. Typically, these might include impurities, degradation products (such as modified analytes, fragments of the analyte, or aggregates of the analyte), matrix components (e.g. from serum), side-products (such as multimers of the analyte) etc.

Multivalent, multispecific antibodies specifically bind to different targets, most likely with different affinities and complex stabilities for each target. Only a fully active multivalent, multispecific antibody can bind to all targets and shows the full biological activity in a corresponding assay.

The term "valency" denotes the number of different binding sites of an antibody for an antigen. A monovalent antibody comprises one binding site for an antigen. A bivalent antibody comprises two binding sites for the same antigen.

An assay design involving first the binding of the multispecific molecule (antibody) to immobilized target #1 (its first antigen), and second the recruiting of target #2 (its second antigen) from solution to the target #1/multispecific molecule complex (antigen#1/multispecific antibody complex) covers both binding events with one activity readout (bridging assay, bridging binding signal).

Potential side-products of multispecific molecules are multimeric forms of the molecule, such as e.g. dimers, trimers, tetramers and higher order aggregates. If the multimeric form can still bind to both targets, the binding to each target is polyvalent rather than monovalent (= functional multimer).

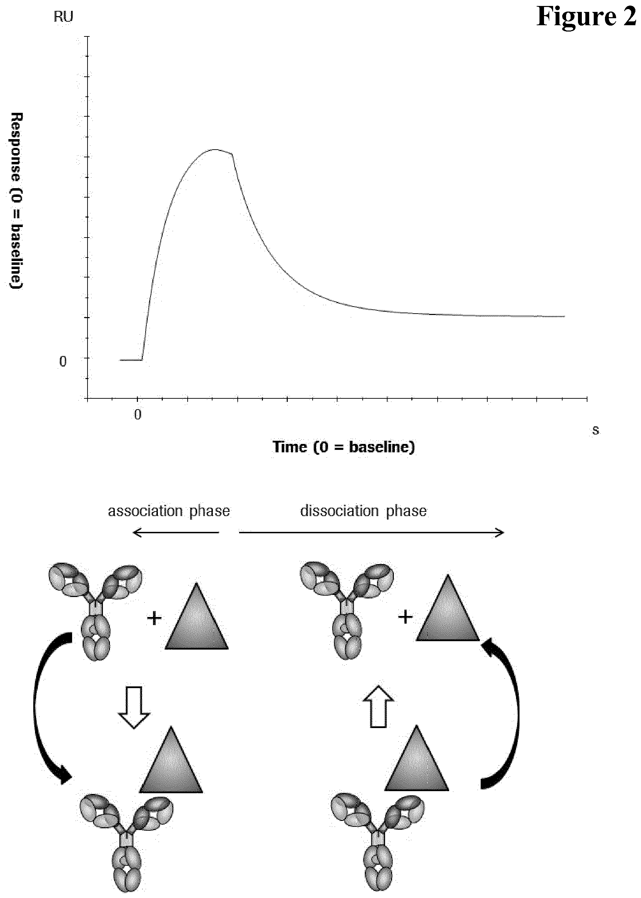

The term "binding affinity" denotes the strength of the interaction of a single binding site with its respective target. Experimentally, the affinity can be determined e.g. by measuring the kinetic constants for association (k_{A}) and dissociation (k_{D}) of the antibody and the antigen in the equilibrium (see Figure 2).

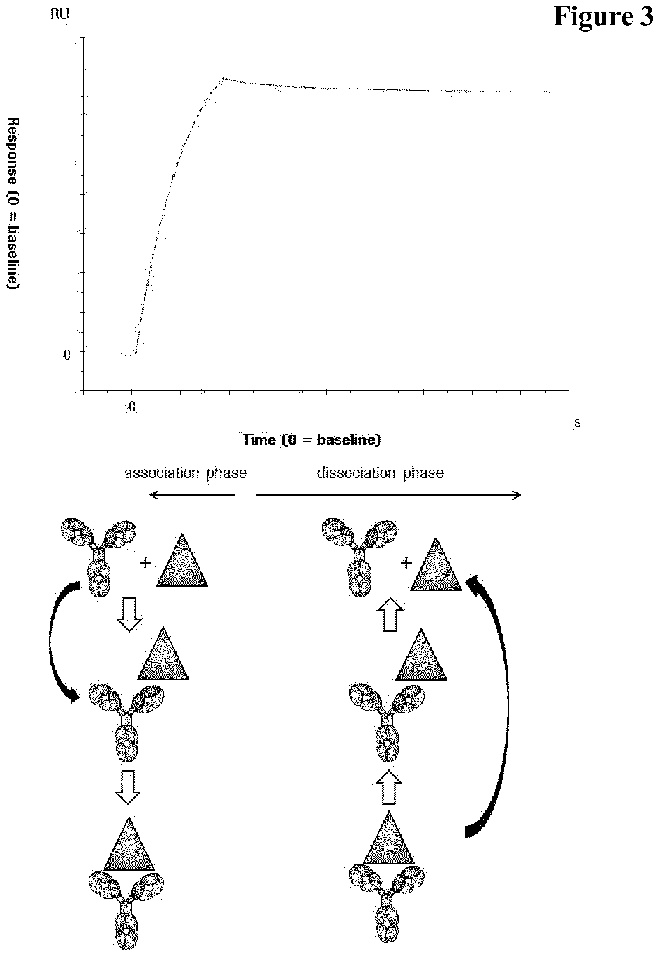

The term "binding avidity" denotes the combined strength of the interaction of multiple binding sites of one molecule (antibody) with the same target. As such, avidity is the combined synergistic strength of bond affinities rather than the sum of bonds (see Figure 3). Requisites for avidity are:
- polyvalency of a molecule, such as an antibody, or of a functional multimer to one target (antigen),
- multiple accessible epitopes on one soluble target OR multiple binding of an antibody to one epitope each on various immobilized targets.

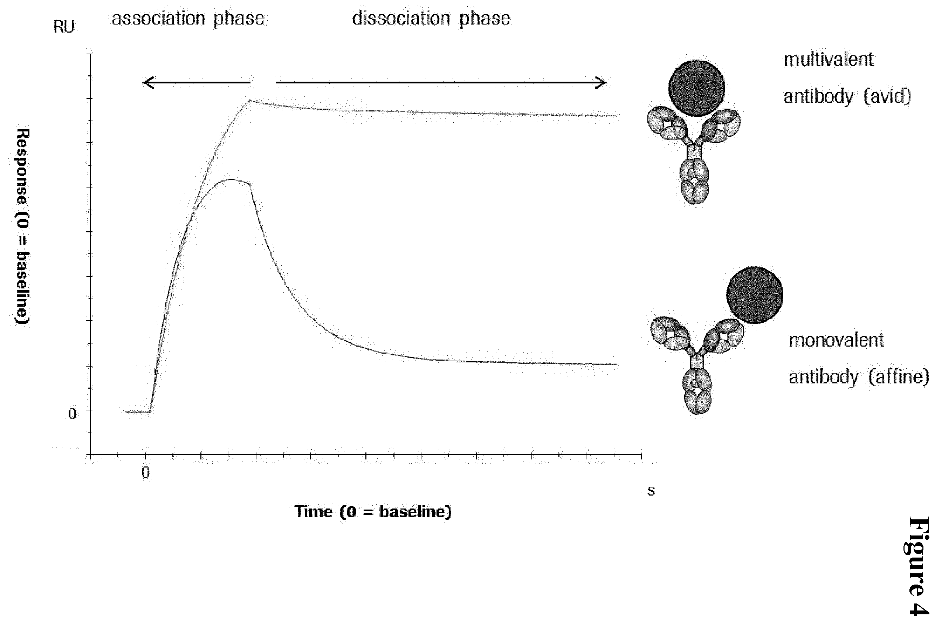

The complex association does not differ between affine and avid binding. However, the complex dissociation for avid binding depends on the simultaneous dissociation of all binding sites involved (see Figure 4). Therefore, the increase of binding strength due to avid binding (compared to affine binding) depends on the dissociation kinetics/complex stability:
- the bigger (higher) the complex stability, the less likely is the simultaneous dissociation of all involved binding sites; for very stable complexes, the difference of affine vs. avid binding becomes essentially zero;
- the smaller (lower) the complex stability, the more likely is the simultaneous dissociation of all involved binding sites; the difference of affine vs. avid binding is increased.

It has been found that functional multimers (e.g. dimers) of multivalent, multispecific antibodies (e.g. bivalent, bispecific antibodies) that are present in preparations containing such antibodies influence the result of an assay for the determination of the biological activity of the multivalent, multispecific antibody (see Figure 1). The functional multimers (dimers) are responsible for non-correct (bridging) binding signal readouts of assays for determining the biological activity of a multivalent, multispecific antibody if certain framework rules as reported herein are not employed.

It has been found that an assay (e.g. an SPR-based assay) for determining the biological activity of a multivalent, multispecific antibody (e.g. a bivalent, bispecific antibody) is not affected by the presence of functional multimers (dimers) of the multivalent, multispecific antibody if:
i) two (different) binding functionalities/specificities of the multivalent, multispecific antibody are simultaneously employed in/used for generating the assay readout/signal (bridging binding signal),
ii) for antibody capture the antigen with the more stable interaction with the antibody is used (i.e. the antigen with the more stable interaction with the antibody or vice versa the binding specificity of the antibody that has the smaller k_{D} value (dissociation constant) for the interaction with its antigen is used for the immobilization/capture of the antibody whereby this antigen is immobilized to the solid phase), resulting in a high antigen/antibody complex stability and, thus, reduced/abolished sensitivity to functional multimers,
iii) as readout the bridging binding signal, i.e. the signal generated by the binding of the antibody to both two antigens, is used, as this signal depends on the simultaneous interaction of the antibody with both antigens.

In more detail, like the monomeric multivalent, multispecific antibody, functional multimers (dimers) bind both targets with different complex stabilities. Functional multimers (dimers) are per definition multivalent (bivalent).

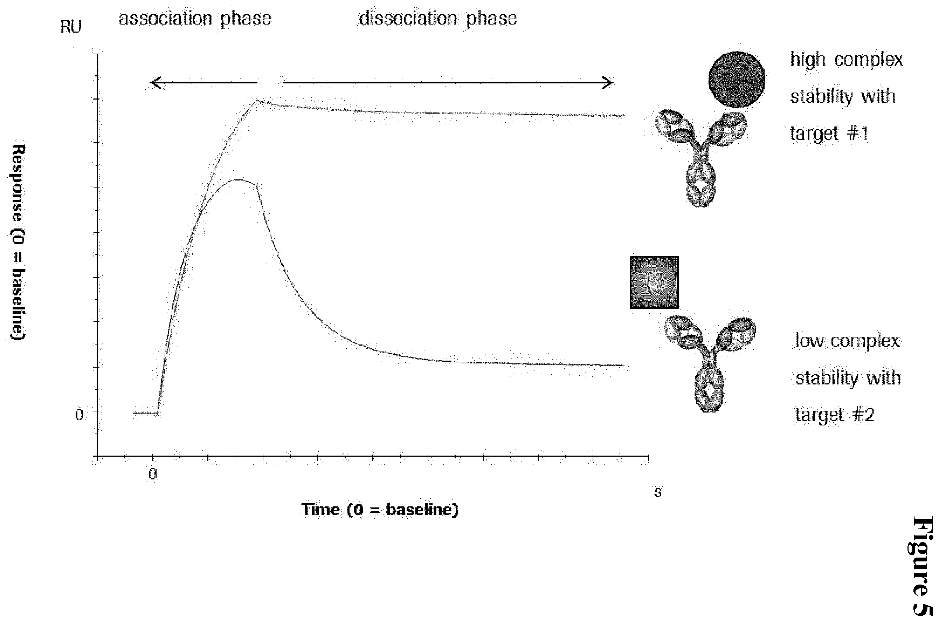

An assay for determining the biological activity of a multivalent, multispecific antibody using a bridging format can be set up using two different orientations: Either target #1 or target #2 can be immobilized on the surface and the second not immobilized target is recruited from solution to the target/multispecific molecule complex. Depending on the strength of the interaction and therewith on the stability of the formed complex the assay readout differs (see Figure 5).

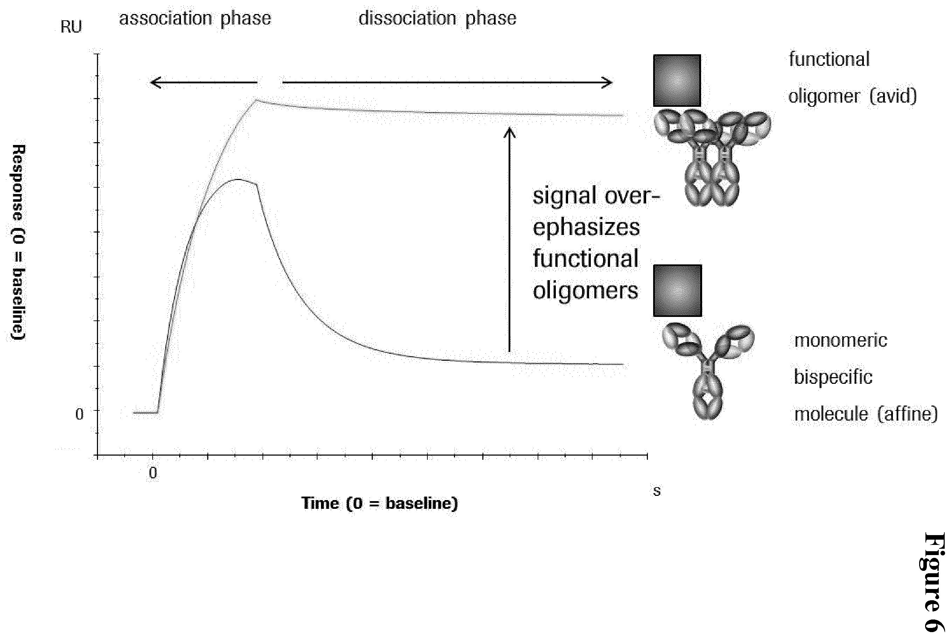

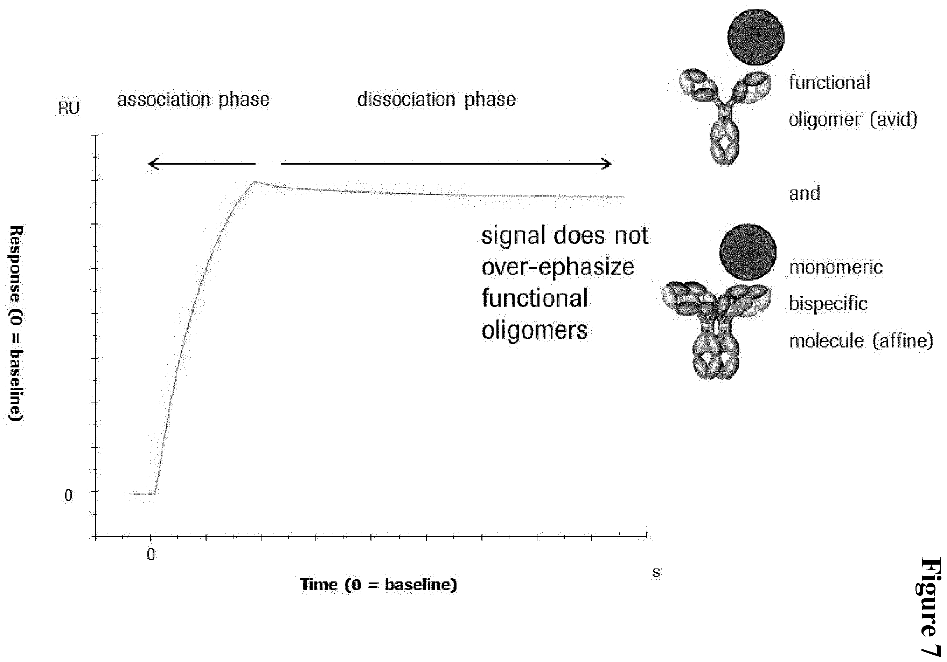

It has been found that the assay setup has a strong impact on the specificity and robustness of an assay for determining the biological activity of a multivalent, multispecific antibody in the presence of functional multimers:
- if the interaction with the smaller (lower) complex stability is used for the capture of the antibody, i.e. is on the surface, the impact of avidity-driven binding relative to affinity-driven binding becomes high; this over-emphasizes functional multimers (e.g. dimers), and the assay is less specific for the multivalent, multispecific antibody or not suitable at all; this can be shown by analyzing mixtures of multispecific molecules and functional multimers: in this case the readout of the biological activity is over-proportional (i.e. 1% multimer results in an increase of more than (>) 1% biological activity detected); (see Figure 6);
- if the interaction with the bigger (higher) complex stability is used for the capture of the antibody, i.e. is on the surface, the impact of avidity-driven binding relative to affinity-driven binding becomes low; this does not over-emphasize functional multimers, and the assay is more specific for the multivalent, multispecific antibody; in this case the readout of the biological activity is proportional (i.e. 1% multimer results in an increase of approx. 1% biological activity detected); (see Figure 7).

It has been found that low complex stability used for the capture of the antibody, i.e. on the surface, results in an assay signal that over-emphasizes functional multimers.

It has been found that high complex stability used for the capture of the antibody, i.e. on the surface, results in an assay signal that does not over-emphasizes functional multimers.

In addition to the readout with respect to the biological activity of the multivalent, multispecific antibody also the individual biological activity of the multivalent, multispecific antibody to the immobilized antigen can be determined.

Additionally to the readout with respect to the biological activity of the multivalent, multispecific antibody also the individual biological activity of the multivalent, multispecific antibody to the bridging antigen can be determined assuming that modifications affecting the binding to any of the antigens occur independently from each other and assuming that all antibodies bind to both antigens.

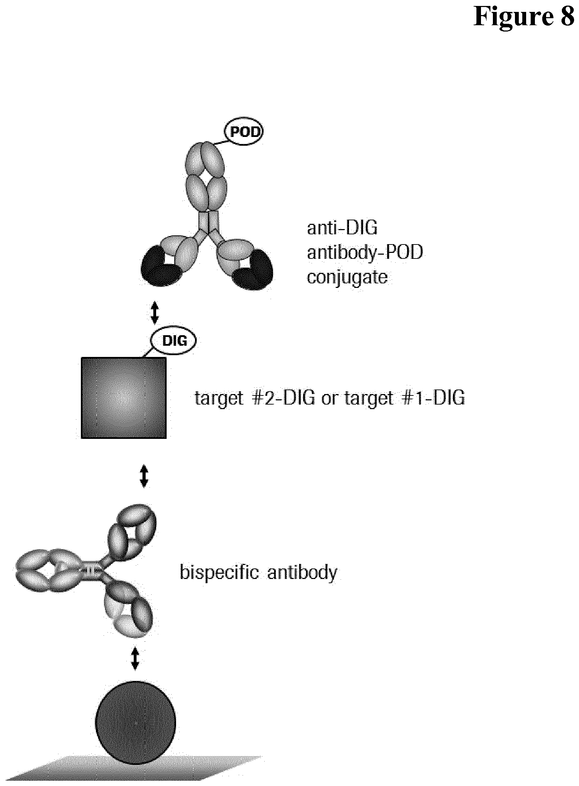

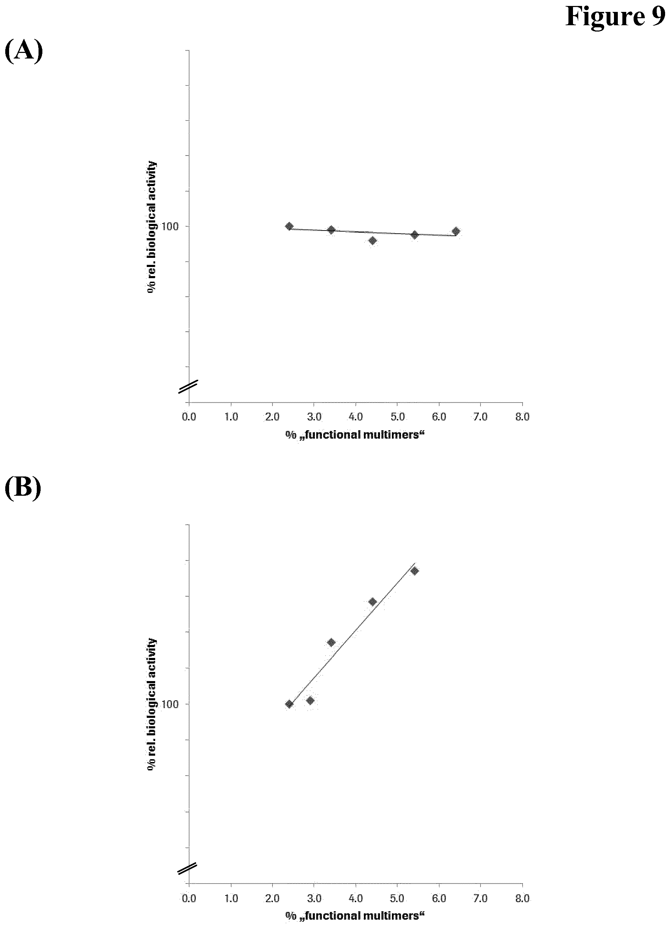

In Figure 9 an exemplary comparison of the results of an ELISA assay (setup shown in Figure 8) depending of the assay setup with increasing concentration of functional multimers is shown (the samples have been obtained by spiking of functional multimers into samples of a bivalent, bispecific antibody). The interaction of the multivalent, multispecific antibody with target#1 is stronger than with target#2 (smaller k_{D} value (dissociation constant) for interaction with target#1 than for interaction with target#2). It can be seen that assay orientation with target#2 immobilized (used as capture agent) displays an increased sensitivity to functional multimers (slope bigger than 1), whereas the orientation with target #1 immobilized does not shown variance with increasing concentration of functional multimer.

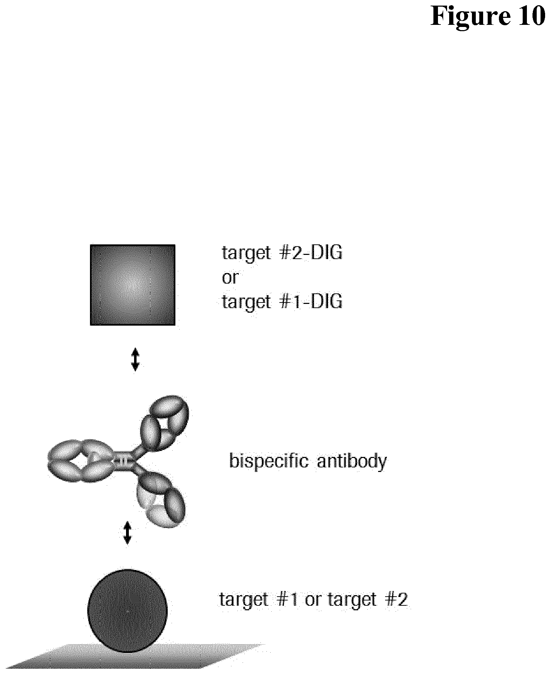

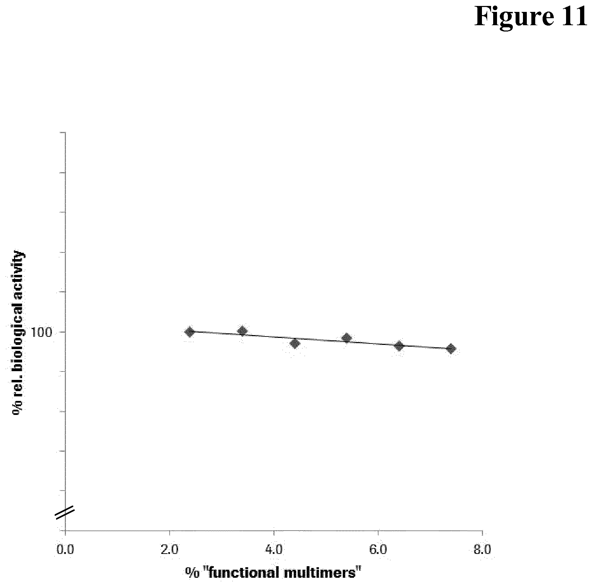

In Figure 11 an exemplary comparison of the results of an SPR-based assay (set-up shown in Figure 10) depending of the assay setup with increasing concentration of functional multimers is shown (the samples have been obtained by spiking of functional multimers into samples of a bivalent, bispecific antibody). The interaction of the multivalent, multispecific antibody with target#1 is stronger than with target#2 (smaller k_{D} value (dissociation constant) for interaction with target#1 than for interaction with target#2). It can be seen that assay orientation with target#1 immobilized does not shown variance with increasing concentration of functional multimer.

The use of SPR allows the determination of the biological activity in real-time.

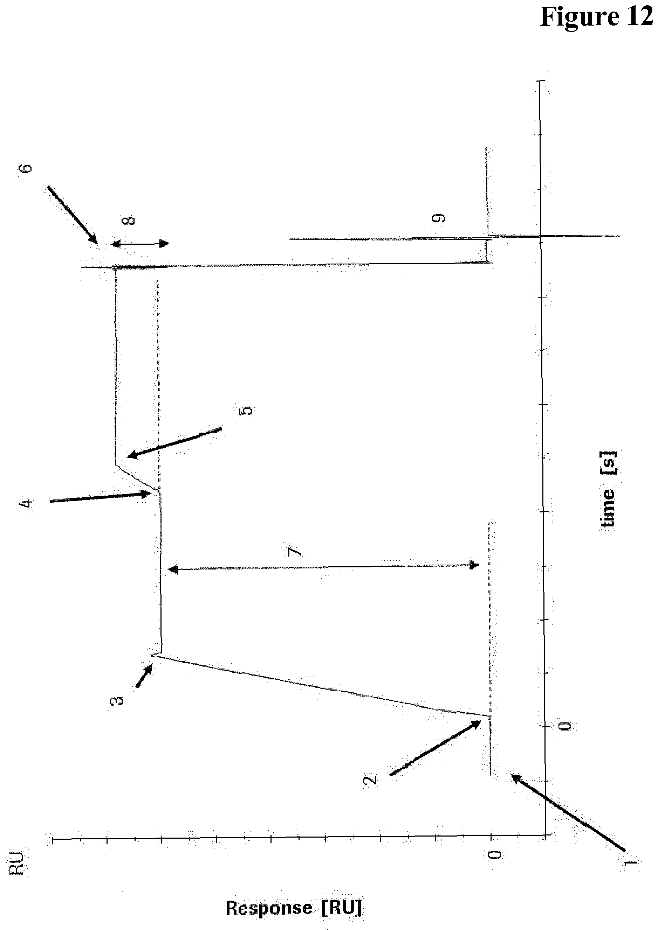

The bridging assay as such and the use of the underlying findings as reported herein in an assay for determining the biological activity of a multivalent, multispecific antibody is based on the effect that by using the interaction with higher complex stability, i.e. smaller k_{D} value (dissociation constant), for capture of the antibody the assay is less sensitive to functional multimers (dimers, trimers, tetramers, or oligomers). The total readout (= bridging binding signal; Figure 12, signal R2 (8)) depends on both interactions of the multivalent, multispecific antibody and covers two functionalities of the multivalent, multispecific antibody. In case of the use of SPR the sensograms harbor an additional readout besides the bridging signal: the binding signal to the immobilized antigen gives the opportunity to quantify molecules which are able to bind the immobilized first antigen (Figure 12, signal R2 (8)). Relative binding signals for all concentrations can be calculated and the average relative response can be used to assess the binding activity of the multivalent, multispecific antibody to its first antigen. In this manner, it is possible to obtain the individual activity to the first antigen en passant to the overall binding activity.

The following examples and figures are provided to aid the understanding of the present invention, the true scope of which is set forth in the appended claims.

### Figures

- **Figure 1**: Influence of the presence of functional dimers (as example of functional multimers) of a bivalent, bispecific antibody on the assay result.
- **Figure 2**: Antibody affinity; white arrows: order of events; black arrows: readout is based on this difference.
- **Figure 3**: Antibody avidity; white arrows: order of events; black arrows: readout is based on this difference.
- **Figure 4**: Comparison of antibody affinity and antibody avidity.
- **Figure 5**: Comparison of antibody interactions with low and high complex stability (high and low k_{D} value).
- **Figure 6**: Low complex stability on the surface results in an assay signal that over-emphasizes functional oligomers.
- **Figure 7**: High complex stability on the surface does not over-emphasizes functional oligomers.
- **Figure 8**: Schematic ELISA bridging assay.
- **Figure 9**: Comparison of influence of spiking of a bivalent, bispecific antibody with functional multimers on the assay orientation in an ELISA; (A) target #1 immobilized; (B) target #2 immobilized.
- **Figure 10**: Schematic SPR-based bridging assay.
- **Figure 11**: Influence of spiking of a bivalent, bispecific antibody with functional multimers in an SPR-based assay.
- **Figure 12**: Obtaining the bridging signal from sensograms; 1: first antigen present and immobilized to sensor surface; 2: bivalent, bispecific antibody binding start; 3: bivalent, bispecific antibody binding stop; 4: second antigen binding start; 5: second antigen binding stop; 6: report point bridging signal; 7: R1; 8: R2; 9: regeneration of sensor chip.
- **Figure 13**: Sensograms can be translated into concentration-based functions; (A) sensograms overlay determined for 0.35 - 30 µg/ml bispecific, bivalent antibody (1:1.5 dilution series); (B) bridging signal (R2) (second antigen); (C) signal (R1) (first antigen).

### Example 1

### General assay setup

### Surface plasmon resonance-based assay (SPR-based assay)

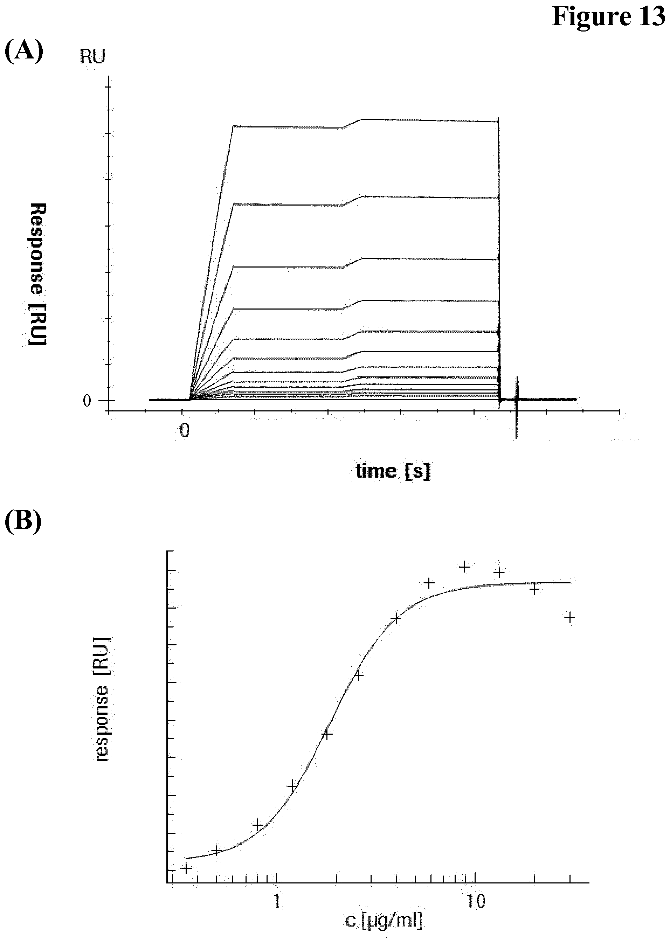

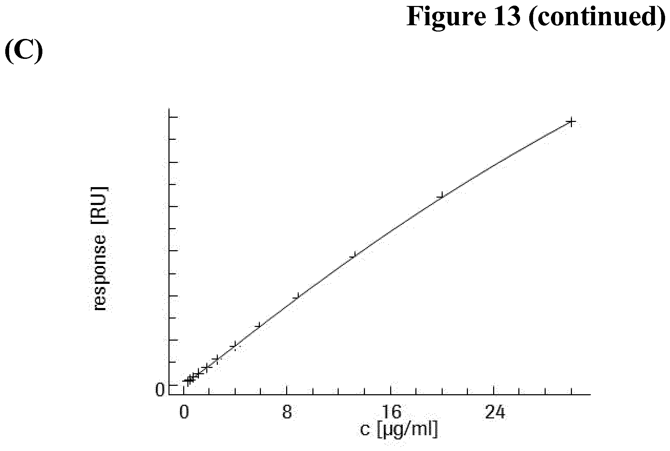

The method is based on an SPR-based assay using BIAcore technology (GE Healthcare). In a first step, VEGF was immobilized to a CM5 sensor chip. Second, the anti-ANG2/VEGF bivalent, bispecific antibody was injected within a defined concentration range. Third, the human ANG2 (receptor binding domain RBD) was injected. The binding responses (resonance units, RU, see Figure 12) obtained after injection of human ANG2 correlate with the amount of anti-ANG2/VEGF antibody bound to both VEGF and ANG2 (the ANG2-related bridging signal R2 (8) is the base for biological activity calculation; the VEGF binding signal R1 (7) is not considered; see Figure 12) and were plotted against the antibody concentration range used (see Figure 13). The resulting linear plot was analyzed by appropriate computer software (e.g. XLfit4, IDBS Software), which fits a 2-parametric line and hence allows determination of the y-axis intercept as the biological activity readout.

### Enzyme-linked immunosorbent assay (ELISA)

In the first step, human ANG2 was immobilized on a micro titer plate (Nunc Maxisorb-MTP). Second, the anti-ANG2/VEGF bivalent, bispecific antibody was added to the immobilized human ANG2 within a defined concentration range. Third, a constant amount of human VEGF was added. Fourth, a complex of mouse anti-VEGF antibody and goat anti-mouse IgG antibody-POD conjugate (POD = horseradish peroxidase) was added after pre-incubation. Finally, 2,2'-azino-bis(3-ethylbenzthiazoline-6-sulphonic acid, ABTS) was added and after a defined time the reaction was stopped by the addition of phosphoric acid. The chromogenic ABTS signal was measured by an ELISA reader. The absorbance signals correlate with the amount of the bivalent, bispecific antibody bound to both human ANG2 and human VEGF and were plotted against the antibody concentration used. This sigmoidal plot was analyzed by XLfit4 (IDBS Software), which fits a 4-parametric logistic curve and hence allows determination of the EC₅₀ value as the biological activity readout.

### Example 2

### Binding properties of bivalent, bispecific ant-ANG2/VEGF antibody

### Binding properties characterized by Surface Plasmon Resonance (SPR) Analysis

Simultaneous binding of both antigens was confirmed by applying Surface Plasmon Resonance (SPR) using a BIAcore T100 instrument (GE Healthcare Biosciences AB, Uppsala, Sweden). VEGF was immobilized to a CM5 Sensor chip using standard amine coupling chemistry. In a first step, the bivalent, bispecific antibody was injected at a concentration of 10 µg/ml in HBS buffer (10 mM HEPES, 150 mM NaCl, 0.05% Tween 20, pH 7.4) at 25°C. After binding of the antibody to the immobilized VEGF, human ANG2 was injected at 10 µg/ml in a second step.

In a further experiment the affinity and binding kinetics of the bivalent bispecific antibody were determined. Briefly, goat anti-huIgG-Fcgamma polyclonal antibodies were immobilized on a CM4 chip via amine coupling for presentation of the bispecific antibody against ANG2 and VEGF. Binding was measured in HBS buffer at 25°C or 37°C. Purified ANG2-His or VEGF was added in various concentrations between 0.37 nM and 30 nM or between 3.7 nM and 200 nM in solution. Association was measured by an injection of 3 minutes; dissociation was measured by washing the chip surface with HBS buffer for 10 minutes and a K_{D} value was estimated using a 1:1 Langmuir binding model. Due to heterogeneity of the ANG2 preparation no 1:1 binding could be observed. Therefore K_{D} values are apparent values. The determined affinity of the bivalent, bispecific antibody to VEGF was extremely high, the calculated off-rate was out of BIAcore specifications even at 37°C. In Table 1 the off-rates for both antigens are summarized.

**Table 1: Kinetic parameters of binding to ANG2 and VEGF**

| **analyte** | **apparent k_{D} (1/s)** |
|---|---|
| ANG2 | 6.3^{∗}10⁻⁰⁴ |
| VEGF | < 1^{∗}10⁻⁰⁶ |

### Assay for quantification of binding active bivalent, bispecific antibody

Additionally to the SPR-based analysis, an ELISA was established to determine the binding of active bivalent, bispecific antibody. In this assay, human ANG2 is directly coated to the wells of a Maxisorb microtiter plate (MTP) in the first step. Meanwhile, the samples/reference standards (bivalent, bispecific antibody) were pre-incubated in the wells of another MTP with digoxigenylated VEGF. After pre-incubation and coating, excess of unbound ANG2 was removed by washing the ANG2 coated MTP. The pre-incubated mixture of bivalent, bispecific antibody and VEGF-Dig was then transferred to the human ANG2 coated MTP and incubated. After incubation, the excess of pre-incubation solution was removed by washing followed by incubation with a horse-radish peroxidase labeled anti-digoxigenin antibody. The antibody-enzyme conjugate catalyzes the color reaction of the ABTS® substrate. The signal was measured by ELISA reader at 405 nm wavelength (reference wavelength: 490 nm ([405/490] nm)).

## Claims

1. Use of the simultaneous binding of a bivalent, bispecific antibody to its first and second antigen in a surface plasmon resonance or ELISA method, whereby the first antigen is immobilized as capture reagent on a solid surface, and whereby the bivalent, bispecific antibody has a k_{D} value for the interaction with the first antigen that is smaller than the k_{D} value for the interaction with the second antigen, for reducing the interference of functional multimeric forms of the bivalent, bispecific antibody in the surface plasmon resonance or ELISA method.

2. The use according to claim 1, wherein the solid surface is a surface plasmon resonance chip.

3. The use according to any one of claims 1 to 2, wherein the first antigen is a dimer or trimer or tetramer.

4. The use according to any one of claims 1 to 3, wherein the functional multimer is a functional dimer.

5. The use according to any one of claims 1 to 4, wherein the k_{D} value of the binding site that has the higher k_{D} value for the interaction with its antigen is at least 1.1 times higher than the k_{D} value of the binding site of the bivalent, bispecific antibody that has the smaller k_{D} value.

6. The use according to claim 5, wherein the k_{D} value is at least 10 times higher.

7. The use according to any one of claims 5 to 6, wherein the k_{D} value is at least 100 times higher.

8. A method for determining the presence of functional multimers of a bivalent, bispecific antibody in a sample, comprising the step of
- comparing the binding signal determined for the bivalent, bispecific antibody using an assay wherein a first antigen is immobilized and used for the capture of the bivalent, bispecific antibody with the binding signal determined for the bivalent, bispecific antibody using the same assay wherein the second antigen is immobilized and used for the capture of the bivalent, bispecific antibody,
whereby the presence of functional multimers of the bivalent, bispecific antibody is determined if the determined binding signal differ by more than the standard deviation of the performed assay.

9. The method according to claim 8, wherein the determining the binding signal for the simultaneous binding of the bivalent, bispecific antibody to its first and second antigen is based on the binding of the bivalent, bispecific antibody to its second antigen.

10. The method according to any one of claims 8 to 9, wherein the solid surface is a surface plasmon resonance chip.

11. The method according to any one of claims 8 to 10, wherein the first antigen is a dimer or trimer or tetramer.

12. The method according to any one of claims 8 to 11, wherein the functional multimer is a functional dimer.

## Patentansprüche

1. Verwendung des simultanen Bindens eines bivalenten, bispezifischen Antikörpers an sein erstes und zweites Antigen in einem Oberflächen-Plasmonresonanz- oder ELISA-Verfahren, wobei das erste Antigen als Fängerreagenz auf einer festen Oberfläche immobilisiert ist, und wobei der bivalente, bispezifische Antikörper einen k_{D}-Wert für die Wechselwirkung mit dem ersten Antigen aufweist, der kleiner ist als der k_{D}-Wert für die Wechselwirkung mit dem zweiten Antigen, zur Verringerung der Interferenz funktioneller multimerer Formen des bivalenten, bispezifischen Antikörpers in dem Oberflächen-Plasmonresonanz- oder ELISA-Verfahren.

2. Verwendung nach Anspruch 1, wobei die feste Oberfläche ein Oberflächen-Plasmonresonanz-Chip ist.

3. Verwendung nach einem der Ansprüche 1 bis 2, wobei das erste Antigen ein Dimer oder Trimer oder Tetramer ist.

4. Verwendung nach einem der Ansprüche 1 bis 3, wobei das funktionelle Multimer ein funktionelles Dimer ist.

5. Verwendung nach einem der Ansprüche 1 bis 4, wobei der k_{D}-Wert der Bindungsstelle, die den höheren k_{D}-Wert für die Wechselwirkung mit ihrem Antigen aufweist, mindestens 1,1-fach höher ist als der k_{D}-Wert der Bindungsstelle des bivalenten, bispezifischen Antikörpers, die den kleineren k_{D}-Wert aufweist.

6. Verwendung nach Anspruch 5, wobei der k_{D}-Wert mindestens 10-fach höher ist.

7. Verwendung nach einem der Ansprüche 5 bis 6, wobei der k_{D}-Wert mindestens 100-fach höher ist.

8. Verfahren zur Bestimmung der Gegenwart funktioneller Multimere eines bivalenten, bispezifischen Antikörpers in einer Probe, umfassend den Schritt
- Vergleichen des Bindungssignals, das für den bivalenten, bispezifischen Antikörper unter Verwendung eines Tests, in dem ein erstes Antigen immobilisiert und zum Fangen des bivalenten, bispezifischen Antikörpers verwendet wird, bestimmt wurde, mit dem Bindungssignal, das für den bivalenten, bispezifischen Antikörper unter Verwendung desselben Tests, in dem das zweite Antigen immobilisiert und zum Fangen des bivalenten, bispezifischen Antikörpers verwendet wird, bestimmt wurde,
wobei die Gegenwart funktioneller Multimere des bivalenten, bispezifischen Antikörpers bestimmt wird, wenn sich das bestimmte Bindungssignal um mehr als die Standardabweichung des durchgeführten Tests unterscheidet.

9. Verfahren nach Anspruch 8, wobei das Bestimmen des Bindungssignals für das simultane Binden des bivalenten, bispezifischen Antikörpers an sein erstes und zweites Antigen auf dem Binden des bivalenten, bispezifischen Antikörpers an sein zweites Antigen basiert.

10. Verfahren nach einem der Ansprüche 8 bis 9, wobei die feste Oberfläche ein Oberflächen-Plasmonresonanz-Chip ist.

11. Verfahren nach einem der Ansprüche 8 bis 10, wobei das erste Antigen ein Dimer oder Trimer oder Tetramer ist.

12. Verfahren nach einem der Ansprüche 8 bis 11, wobei das funktionelle Multimer ein funktionelles Dimer ist.

## Revendications

1. Utilisation de la liaison simultanée d'un anticorps bispécifique et bivalent à son premier et son second antigène dans une résonance plasmonique de surface ou une méthode ELISA, le premier antigène étant immobilisé comme réactif de capture sur une surface solide, et l'anticorps bispécifique et bivalent ayant une valeur k_{D} pour l'interaction avec le premier antigène qui est inférieure à la valeur k_{D} pour l'interaction avec le second antigène, pour réduire l'interférence de formes multimères fonctionnelles de l'anticorps bispécifique et bivalent dans la résonance plasmonique de surface ou la méthode ELISA.

2. Utilisation selon la revendication 1, dans laquelle la surface solide est une puce de résonance plasmonique de surface.

3. Utilisation selon l'une quelconque des revendications 1 à 2, dans laquelle le premier antigène est un dimère ou un trimère ou un tétramère.

4. Utilisation selon l'une quelconque des revendications 1 à 3, dans laquelle le multimère fonctionnel est un dimère fonctionnel.

5. Utilisation selon l'une quelconque des revendications 1 à 4, dans laquelle la valeur k_{D} du site de liaison qui a la valeur k_{D} la plus élevée pour l'interaction avec son antigène est au moins 1,1 fois plus élevée que la valeur k_{D} du site de liaison de l'anticorps bispécifique et bivalent qui a la valeur k_{D} la plus faible.

6. Utilisation selon la revendication 5, dans laquelle la valeur k_{D} est au moins 10 fois plus élevée.

7. Utilisation selon l'une quelconque des revendications 5 à 6, dans laquelle la valeur k_{D} est au moins 100 fois plus élevée.

8. Procédé de détermination de la présence de multimères fonctionnels d'un anticorps bispécifique et bivalent dans un échantillon, comprenant l'étape de
- comparaison du signal de liaison déterminé pour l'anticorps bispécifique et bivalent en utilisant un dosage dans lequel un premier antigène est immobilisé et utilisé pour la capture de l'anticorps bispécifique et bivalent avec le signal de liaison déterminé pour l'anticorps bispécifique et bivalent en utilisant le même dosage dans lequel le second antigène est immobilisé et utilisé pour la capture de l'anticorps bispécifique et bivalent,
la présence de multimères fonctionnels de l'anticorps bispécifique et bivalent étant déterminée si le signal de liaison déterminé diffère par plus de l'écart-type du dosage réalisé.

9. Procédé selon la revendication 8, dans lequel la détermination du signal de liaison pour la liaison simultanée de l'anticorps bispécifique et bivalent à son premier et son second antigène est basée sur la liaison de l'anticorps bispécifique et bivalent à son second antigène.

10. Procédé selon l'une quelconque des revendications 8 à 9, dans lequel la surface solide est une puce de résonance plasmonique de surface.

11. Procédé selon l'une quelconque des revendications 8 à 10, dans lequel le premier antigène est un dimère ou un trimère ou un tétramère.

12. Procédé selon l'une quelconque des revendications 8 à 11, dans lequel le multimère fonctionnel est un dimère fonctionnel.
